Europäisches Patentamt

European Patent Office

Office européen des brevets

(11) Publication number: **0 256 880 B1**

# EUROPEAN PATENT SPECIFICATION

(45) Date of publication of patent specification: **03.05.95**  (51) Int. Cl.⁶: **C08B 37/10**, A61K 31/725

(21) Application number: **87307292.0**

(22) Date of filing: **18.08.87**

The file contains technical information submitted after the application was filed and not included in this specification

(54) **Alkanoyl esters of heparin of low molecular weight.**

(30) Priority: **20.08.86 US 898124**
**20.08.86 US 898127**
**20.08.86 US 898130**
**20.08.86 US 898431**
**20.08.86 US 898436**

(43) Date of publication of application:
**24.02.88 Bulletin 88/08**

(45) Publication of the grant of the patent:
**03.05.95 Bulletin 95/18**

(84) Designated Contracting States:
**AT BE CH DE ES FR GB GR IT LI LU NL SE**

(56) References cited:
**EP-A- 0 101 141**
**DE-A- 2 128 377**
**US-A- 4 331 697**

**PATENT ABSTRACTS OF JAPAN, vol. 2, no. 8 (C-77), 20th January 1978, page 3840;& JP-A-52 111 983**

(73) Proprietor: **MIAMI UNIVERSITY**
**500 East High Street**
**Oxford**
**Ohio 45056 (US)**

(72) Inventor: **Foley, Kevin Michael**
**5757 Lynn Street**
**Franklin**
**Ohio 45005 (US)**
Inventor: **Griffin, Charles Campbell**
**710 Melissa Drive**
**Oxford**
**Ohio 45056 (US)**
Inventor: **Amaya, Eduardo**
**811 Sands Avenue**
**Monroe**
**Ohio 45050 (US)**

(74) Representative: **Burford, Anthony Frederick et al**
**W.H. Beck, Greener & Co.**
**7 Stone Buildings**
**Lincoln's Inn**
**London WC2A 3SZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

## Description

The present invention is directed to $C_2$-$C_{10}$ alkanoyl ester derivatives of low molecular weight heparin which exhibit improved anti-Xa activity in relation to global anticlotting activity.

The chemical structure of heparin is complex. Heparin is not a single compound, but rather is a mixture of compounds. However, heparin is commonly thought to primarily be a polymeric substance made up of tetrasaccharide repeating units. On the average each tetrasaccharide repeating unit contains approximately 5 free hydroxyl groups and has a molecular weight of approximately 1229. The average molecular weight of commercially available heparin varies from about 10,000 to about 18,000 daltons. Accordingly, on the average, commercially available heparin contains approximately 8 to 15 tetrasaccharide repeating units.

The term "heparin" is used in the specification and the claims in its broadest sense, in order to designate either a commercial heparin of pharmaceutical grade or a crude heparin such as obtained by extraction from biological material, particularly from mammalian tissue. It also includes mucopolysaccharides exhibiting anticoagulant properties that are synthesized from non-heparin sources.

The term "low molecular weight heparin" is used in the specification in its broadest sense, in order to designate a low molecular weight fraction isolated from heparin, a product obtained by depolymerizing heparin, or mucopolysaccharides exhibiting anticoagulant properties that are synthesized from non-heparin sources. "Low molecular weight" is used to mean a material exhibiting a molecular weight of less than 10,000 daltons.

Heparin is the most widely used agent for immediate management of most thromboembolic disorders, particularly, deep vein thrombosis and pulmonary and systemic emboli. An important problem is that the dosage must be balanced in such a manner that good thrombosis protection is obtained while bleeding complications are avoided. In many instances bleeding or hemorrhaging has been a major problem, some investigators reporting the incidence of hemorrhaging to be as high as 35 percent.

Heparin functions to block the coagulation cascade at various sites by interfering with a multiplicity of blood factors including factor Xa. Anti-Xa activity which is accompanied by little global anticoagulant activity is indicative of strong antithrombotic activity while avoiding the risk of hemorrhage. It should be noted that heparin simultaneously depresses a large number of the coagulation factors participating in the creation and the maintenance of different forms of hypercoagulability. Thus, heparin's activity appears to be global rather than specific.

APTT and USP anticoagulant assays are recognized as measuring global anticoagulant activity. We prefer to use the APTT assay to measure global anticoagulant activity.

Heparin is currently the medication of choice for preventing the risk of hypercoagulation, for example the appearance of postoperative thrombosis. However excessive amounts of heparin may be at the origin of serious hemorrhages. Hence, considerable care must be exercised to utilize the proper amount of heparin to prevent hypercoagulation without utilizing a sufficient amount to cause hemorrhages. Hence, it is necessary to keep a constant watch on the patient and adjustments in heparin administration must be made depending on the results of blood coagulation tests which must be administered at regular intervals.

One object of this invention is to provide medicaments which overcome the difficulties and constant testing that is associated with heparin administration. This is accomplished by providing compounds that are much more effective than heparin in preventing the risk of hypercoagulation when compared to the associated side effect of causing hemorrhages. The prior art related to heparin derivatives and low molecular weight heparin derivatives possessing modified Anti-Xa specificity is set forth immediately below:

US-A- 4,281,108 discloses a process for obtaining low molecular weight heparin comprising acidification of heparin, depolymerization in the presence of peroxides, and sulfation. The molecular weights of the products are 4,000 to 12,000 daltons. An anti-Xa/APTT ratio greater than 1 is claimed for the products of this patent.

US-A- 4,303,651 teaches the depolymerization of heparin with nitrous acid or by periodate oxidation to produce low molecular weight heparin fragments exhibiting improved inhibition of activated factor X. Said low molecular weight heparin fragments contain 14-18 sugar units.

US-A- 4,351,938 discloses a process for the preparation of heparin derivatives exhibiting an improved anti-Xa value. Said heparin derivatives exhibit a molecular weight of 2,000-7,000 daltons (compared to the molecular weight of commercial heparin of 10,000 to 25,000 daltons) and possess analyzable reducing end groups of which the majority are anhydromannose.

US-A- 4,396,762 discloses a heparin product obtained by degradation of heparin with heparinase from Flavobacterium heparinum (ATCC 13125) or mutants thereof having activity to reduce the coagulation activity of factor X while not effecting the coagulation activity of thrombin.

US-A- 4,401,662 discloses oligosaccharides obtainable from heparin, said oligosaccharides comprising not more than 8 saccharide units one of which is an N-sulfate-3-O-sulfate- D-glucosamine unit. These oligosaccharides may be separated from heparin by means of gel filtration and exhibit a highly selective activity against activated factor X (factor Xa). This results in a strong antithrombotic activity while avoiding the risk of hemorrhage for the patient.

US-A- 4,401,785 teaches a process for producing oligosaccharides having a highly selective activity against activated factor X (factor Xa) of blood i.e. a strong antithrombotic activity while avoiding the risk of hemorrhage. Said process involves depolymerizing heparin and separating the desired oligosaccharides by contacting with AT III (antithrombin III) followed by a subsequent separation of the desired oligosaccharides from AT III.

US-A- 4,415,559 discloses an anticoagulant containing heparin having low antithrombin III affinity as an effective ingredient and which provides a reduced danger of hemorrhage. The low antithrombin III affinity heparin is separated from commercial heparin by affinity chromatography utilizing a gel lattice to which is bonded antithrombin III. The desired heparin fraction is not absorbed by the lattice-bound antithrombin III gel.

US-A- 4,438,108 describes a mixture of oligo- and polysaccharides having an improved antithrombotic activity vs. hemorrhagic activity as compared to heparin. The product described in this patent can be liberated from mammalian tissue by autolysis or with the aid of proteolytic enzymes followed by isolation using organic solvents, quaternary aliphatic ammonium compounds and/or a basic ion exchanger.

US-A- 4,438,261 discloses chemically partially depolymerized heparin having a molecular weight of from about 2000 to 7000 daltons and having analyzable reducing end groups of which the majority are anhydromannose groups. This product exhibits an improved therapeutic index which is defined in US-A- 4,438,261 as the ratio of the anti-Xa activity to the USP activity.

US-A- 4,474,770 discloses oligosaccharides obtainable from heparin, said oligosaccharides comprising not more than 8 saccharide units one of which is an N-sulfate-D-glucosamine unit. These oligosaccharides exhibit a high anti-Xa activity relative to heparin while the global coagulation activity relative to heparin is very low. Thus, the oligosaccharides are claimed to be advantageously useful for antithrombotic treatment without hemorrhage risks.

US-A- 4,486,420 discloses heparinic mucopolysaccharide fractions which have improved antithrombotic activity in vivo (measured in terms of activity of anti-Xa per milligram) compared to heparin and which are more selective with respect to anti-Xa activity than heparin. Said fractions have a molecular weight in the range of about 2,000 to 10,000 daltons and are insoluble in alcohol.

US-A- 4,500,519 describes a process for producing mucopolysaccharide heparinic fractions having improved anti-Xa activity compared to heparin. Said fractions are prepared by depolymerizing heparin to a molecular weight range of 2,000 to 8,000 and separating fractions having selected terminal structures.

US-A- 4,533,549 discloses the depolymerization and fractionation of heparin to obtain derivatives of heparin having a molecular weight of from about 2,500 to 4,000 daltons and improved anti-Xa activity relative to global anticoagulant activity.

GB-B- 2,002,406 teaches the sulfation of a low molecular weight heparin having a molecular weight of from 2,600 to 5,500. An improved antithrombotic activity (anti Xa activity) to the anti-blood clotting activity (KCCT activity) is claimed for the products of this invention vs. heparin.

CA-A- 1,195,322 discloses a process for obtaining low molecular weight heparin comprising the steps of acidifying normal heparin, and depolymerizing in the presence of an oxidizing agent to obtain a low molecular weight heparin product. An anti-Xa/APTT ratio of "almost two" is disclosed.

L.O. Andersson et al in THROMBOSIS RESEARCH, Vol. 9, 1976 pages 575-583 discusses fractions of varying molecular weight isolated from heparin. The molecular weights of the fractions varied from 5,000 to 40,000. Anti-Xa and APTT tests were run on the various fractions. In general, the data indicated that the lower molecular weight fractions exhibited higher anti-Xa values in relation to the APTT values and higher molecular weight fractions exhibited lower anti-Xa values in relation to the APTT values.

JP-A-52 111983 discloses that water-soluble heparin derivatives for intravenous administration can be obtained by reaction of heparin with $C_2$-$C_{10}$ fatty acid anhydride in formamide and, preferably, in the presence of pyridine. Heparin is defined to be a sulphatopolysaccharide having a molecular weight from 6,000 to 20,000. It is stated that it is not considered necessary for all OH groups of heparin to form ester groups with the fatty acid anhydride and that, generally, a partially esterified compound is sufficient wherein only a fraction of OH groups is esterified. No quantitative details concerning the extent of esterification are provided. The only exemplified reaction is with acetic anhydride and the resultant product had equivalent activity to the sodium heparin starting material.

US-A-4,331,697 relates to the preparation of heparin esters having active acrylic type double bonds for attachment to a solid substrate.

DE-A-2128377 discloses substantially water-insoluble long chain fatty acid esters of heparin.

EP-A- 0101141 discloses a depolymerized heparin having an anti-Xa/APTT ratio of almost 2.

The prior art substances derived from heparin and having improved anti-Xa activity in relation to global anticlotting activity have been obtained by isolating lower molecular weight fractions from heparin and/or depolymerizing heparin. We have been able to realize a further improvement in anti-Xa activity in relation to global activity by using an entirely different and novel approach.

Unexpectedly, it has been observed that $C_2$-$C_{10}$ alkanoyl ester, especially acetyl, derivatives of low molecular weight heparin exhibit a higher anti-Xa activity in relation to APTT activity than low molecular weight heparin itself. Anti-Xa values are obtained using the Coatest anti-Xa test kit from KabiVitrum AB, Stockholm, Sweden. APTT (Activated Partial Thromboplastine Time) values are obtained following the procedure described in Andersson et al, Thromb. Res. 9, 575 (1976). APTT is a measure of global anticlotting activity.

As mentioned previously, heparin is the most widely used agent for immediate management of most thromboembolic disorders, particularly, deep-vein thrombosis and pulmonary and systemic emboli. Treatment times vary depending upon the use. Deep-vein thrombosis and pulmonary embolism are typically treated for 7-10 days. Thromboembolic disorders in pregnancy are typically treated for 2-6 weeks. Coronopathies, myocardiopathies, myocardial infraction and angina pectoris are typically treated for 30 days to many months. Heparin must be administered by injection or intravenous infusion (parenteral). It is well known that commercial heparin is not capable of crossing the barrier posed by cell membranes such as those found in the intestine and cannot, therefore be an effective therapeutic agent when administered orally or rectally, for example.

The only commercially successful anticoagulant capable of oral administration is warfarin-sodium which is marketed under the trademark "Coumadin, Sodium." See for example US-A-2,999,049. Warfarin-sodium is a widely used rodenticide and is generally recognized as being inferior to heparin as an anticoagulant. Improving the ability of heparin to pass through membranes as, for example, is necessary in the case of heparin administered orally, rectally, transdermally, or topically, has been the subject of numerous patents. However, none of these products and approaches has proven to be commercially or technically successful. The prior art related to improved permeability are set forth immediately below:

US-A- 3,088,868 teaches the use of an amino acid adjuvant in conjunction with heparin to enable the heparin to be absorbed from the gastro-intestinal tract.

US-A- 3,482,014 teaches the conversion of a portion of the ionic sites of heparin to the acid form. This permits absorption through the walls of the intestinal tract.

US-A- 3,506,642 teaches conversion of the commercially available sodium heparin to the acid form followed by complexing with a suitable amino acid. This results in complexes which can be absorbed through the walls of the intestine. US-A-3,577,534, which is a continuation in part of US-A-3,506,642, teaches the use of said complexes in therapeutic compositions wherein the heparin is also absorbed through the walls of the intestine.

US-A- 3,510,561 teaches the preparation of compositions containing heparin and a sulfone. This permits absorption of the heparin through mucous membranes.

US-A- 3,546,338 teaches the combination of heparin, a metabolizable oil, water and a dispersing agent. Said combination is capable of being absorbed in the alimentary canal of mammals.

US-A- 3,548,052 teaches the use of alkyl sulfoxides, such as dimethyl sulfoxide, in conjunction with heparin to promote the absorption of heparin through mucous membranes.

US-A- 3,574,831 teaches the preparation of compositions containing sodium taurocholate and heparin. These compositions can be absorbed through the walls of the alimentary canal when administered orally or rectally.

US-A- 3,574,832 teaches compositions containing heparin and a surfactant selected from sodium lauryl sulfate, dioctyl sodium sulfosuccinate, sodium hexyl sulfate, sodium lauryl sulfonate, sodium cetyl sulfonate and mixtures thereof.

US-A- 3,835,112 teaches the preparation of heparin esters derived from fatty acids having at least 16 carbon atoms which can be administered orally. These esters are prepared by reacting the Hyamine 1622 salt of heparin with fatty acids in the presence of a carbodiimide.

US-A- 4,239,754 teaches the use of liposomes with heparin retained therein or thereon. The preparations of this patent are said to be orally active.

US-A- 4,281,108 (see above) claims oral activity for a low molecular weight heparin.

US-A- 4,331,697 teaches the preparation of heparin derivatives containing an active carbon-carbon double bond wherein the active carbon-carbon double bond is utilized to bond heparin to a biomedical material.

US-A- 4,440,926 teaches the preparation of selected heparin esters by reaction at the carboxyl sites of heparin. Said esters are prepared by reacting a quaternary ammonium salt or amine salt of heparin with an alcohol or a halide.

US-A- 4,510,135 discloses the use of organic ammonium heparin complexes for oral activity.

US-A- 4,533,549 (see above) teaches the oral activity of depolymerized and fractionated of heparin. However, the hydrophilic character of the compounds of this patent would be unsatisfactory for effective permeability.

GB-B- 2,002,406 (see above) teaches the oral activity of sulfated low molecular weight heparin.

All of the above approaches suffer from shortcomings with regard to increasing the permeability of heparin. Additives, adjuvants, chemical modifications, and heparin derivatives of the prior art have proven to be unwieldy and ineffective.

It is well known that commercial heparin is not capable of crossing the barrier posed by the cell membranes of the intestine (see for example C. Doutremepuich et al., Path. Biol. 32, 45-48 (1984) or US-A-3,548,052). The tendency of a substance to pass through cell membranes can be measured in terms of a permeability constant (p) which is linearly related to the partition coefficient (r) between a water immiscible liquid and water itself, and inversely related to the square root of the molecular weight (M) of the substance crossing the membrane (see for example J. Diamond and Y. Katz, J. Membr. Biol. 17, 121-154 (1974); J. Danielli, "The Permeability of Natural Membranes", Cambridge University press, Cambridge (1952); A. Kotyk, Biochim. Biophys. Acta 300, 183 (1973)). This is shown in equation form below.

$$P \propto \frac{r}{M^{0.5}}$$

Therefore, in order to increase the permeability constant for heparin, it is necessary to increase its partition coefficient (hydrophobicity) and/or decrease its molecular weight.

It has now been discovered that the permeability of low molecular weight heparin can be significantly increased by the addition of $C_2$ to $C_{10}$ alkanoyl ester groups to low molecular weight heparin. Surprisingly, ester groups containing as few as 2 carbon atoms are effective at significantly increasing the permeability. Preferably, the ester groups contain 3 or more carbon atoms.

In a first aspect the invention provides an enteral or topical pharmaceutical composition comprising a $C_2$-$C_{10}$ alkanoyl ester obtainable by reacting an acid chloride with a low molecular weight heparin having a molecular weight less than 10,000 daltons, said ester containing more than 0.1 ester groups per tetrasaccharide unit, having a butanol/water (3:2) partition coefficient greater than 1 x $10^{-4}$, having a relative permeability, as compared to heparin, of 1.5 or greater, and an anti-Xa/APTT ratio greater than 1.5, and a pharmaceutically acceptable carrier or diluent.

In a second aspect the invention provides a $C_2$-$C_{10}$ alkanoyl ester obtainable by reacting an acid chloride with a heparin having a molecular weight less than 6,000 daltons, said ester containing more than 0.1 ester groups per tetrasaccharide unit, having a butanol/water (3:2) partition coefficient greater than 1 x $10^{-4}$, having a relative permeability, as compared to heparin, of 1.5 or greater, and an anti-Xa/APTT ratio greater than 1.5.

Preferably, alkanoyl groups are butyryl, propionyl, decanoyl or especially acetyl.

It is preferred that the esters have an anti-Xa/APTT ratio greater than 2.8.

We prefer to prepare the esters of the invention by allowing a suitable acid chloride to react with low molecular weight heparin. As is apparent to one skilled in the art, a wide variety of reaction conditions and solvents can be used to effect this reaction. We prefer to use formamide as the solvent and pyridine as the hydrochloric acid scavenger for the reaction. The degree of substitution can be changed by varying the ratio of acid chloride to low molecular weight heparin, by varying the solvent or by using no solvent at all, by varying the reaction time and/or by varying the reaction temperature.

As is apparent to those skilled in the art there are many workup procedures which will allow the isolation of low molecular weight heparin ester derivatives possessing a permeability significantly greater than heparin. We prefer the use of dialysis in the workup procedure to isolate low molecular weight heparin ester derivatives possessing high permeability.

Heparin is a mucopolysaccharide composed of amino sugar and uronic acid residues. Heparin is obtained from beef, porcine, sheep, whale, or other mammalian tissue by extraction via procedures known to those skilled in the art. Commercial heparin preparations are now widely available from many sources and are distributed primarily for use as intravascular anticoagulants.

Heparin preparations are known to be heterogeneous on a molecular level. Thus, they exhibit a considerable degree of polydispersity in molecular size, variations in the ratio of glucuronic acid to iduronic acid, alterations in the amount of ester and N-sulfation, and differing extents of N-acetylation. Changes in these parameters have been correlated only to a very limited extent with heparin's anticoagulant potency.

Heparin utilized in the practice of this embodiment of the invention may be derived from porcine intestinal mucosa, beef lungs, and whale tissue as well as from other sources known to those skilled in the art. Synthetically derived heparin and heparin-like substances may also be utilized in the practice of this invention. The preferred sources for use in this embodiment of the invention are porcine intestinal mucosa and beef lungs.

The products of this embodiment of the invention are solids. They can be readily formulated into powders, pills, lozenges, tablets, capsules, ointments, liquids or other suitable forms. Where the compositions are to be swallowed and absorption is to take place in the intestine, the compositions may be given an enteric coating such as cellulose acetatephthalate, or styrene-maleic anhydride copolymers. Enteric coatings are well known to those skilled in the art and are discussed for example in Remington's Practice of Pharmacy and in US-A-3,126,320.

Preparation of buccal or sublingual tablets and of rectal enemas, suppositories and ointments as well as nasal mists, inhalants and transdermal delivery systems can be easily accomplished.

The following Examples are given by way of illustration only and are not to be considered as limiting of this invention.

The low molecular weight heparin used in the examples below was prepared following CA-A-1,195,322. It exhibited a molecular weight of 5187 daltons. Thus, this low molecular weight heparin contained approximately 4.2 tetrasaccharide repeating units.

The heparin used in the examples below is porcine mucosal heparin manufactured by Hepar Industries, Inc., Franklin, Ohio, U.S.A. It exhibited a molecular weight of 13684 daltons. Thus, this heparin contained approximately 11.3 tetrasaccharide repeating units.

## EXAMPLE 1

2 grams of low molecular weight heparin was added to a 250 milliliter round bottom flask protected from the atmosphere by a drying tube. To this was added 24 milliliters of formamide and 24 milliliters of pyridine. The flask was placed in an oil bath maintained at 40°C. 40 milliliters of acetyl chloride was added slowly over a 3-4 hour period with agitation and agitation continued overnight.

50 milliliters of water was then added with agitation. The contents of the flask were then placed in a 2000 molecular weight cutoff dialysis bag (Spectrum Medical Industries, Los Angeles, CA). Dialysis was conducted against a 1% (w/v) sodium chloride solution for 24 hours. The dialysis against 1% sodium chloride was repeated three times. Dialysis was then conducted against water for 24 hours. The dialysis against water was then repeated three times.

The contents of the dialysis bag were then lyophilized to obtain a dry, white powder.

The product was analyzed for anti-Xa and found to exhibit a value of 16.0 units per milligram. The product also was analyzed for APTT and found to exhibit an APTT value of 4.0 units per milligram. Thus, the anti-Xa to APTT ratio was determined to be 4.0.

The low molecular weight heparin starting material was analyzed for anti-Xa and found to exhibit a value of 88.0 units per milligram. This same low molecular weight heparin was found to have an APTT value of 31.7 units per milligram. Thus, the anti-Xa to APTT ratio was determined to be 2.8.

The infrared spectrum was obtained on the product. An absorption peak was observed at 1743 $cm^{-1}$. This peak is characteristic of an ester group, and was not present in the starting low molecular weight heparin.

The number of ester groups per tetrasaccharide unit contained in the product was measured following the method of S. Hestrin, J. BIOL. CHEM, vol 180, pages 249-261, 1949. Butyryl choline chloride was used as the ester standard. A theoretical formula weight of 1229 was used for a tetrasaccharide unit. The results indicated the presence of 4.4 acetyl groups per tetrasaccharide unit.

0.1 grams of the product was added to 2 milliliters of deionized water. 3 ml of butanol was added. The mixture was mixed well and allowed to undergo freeze/thaw cycles until the top layer (butanol) was clear. The amount of product in each layer was determined by running the uronic acid assay according to E.V. Chandrasekaran and J.N. BeMiller, Methods in Carbohydrate Chemistry," vol. VIII, pages 89-96 (1980) using heparin (porcine intestinal mucosa) standards. The results indicated a butanol/water partition coefficient of $1.0 \times 10^{-3}$. The butanol/water partition coefficient for the low molecular weight heparin was determined in the same manner and indicated to be $0.1 \times 10^{-3}$.

The molecular weight of the product was estimated by taking the sum of the theoretical formula weight of a tetrasaccharide unit (1229) plus 4.4 times the formula weight of an acetyl group less the formula weight of the hydrogen replaced in the heparin (42) and multiplying said sum by the molecular weight of the starting low molecular weight heparin (5187) and finally dividing by the theoretical formula weight of a tetrasaccharide unit (1229). This gave a molecular weight of 5967 for the product.

The permeability of the product relative to heparin (porcine intestinal mucosa) was determined as follows. The butanol/water partition coefficient of the product was divided by the square root of the molecular weight of the product. A corresponding value was then obtained for heparin. The value obtained for the product was then divided by the corresponding value for heparin to give 15.1 which is the permeability relative to heparin. This compares with a value of 1.6 determined for the low molecular weight heparin using the same method.

EXAMPLE 2

The procedures of Example 1 were repeated except that the oil bath was maintained at 30°C and 20 milliliters of butyryl chloride added instead of the acetyl chloride to obtain a dry, white powder.

The anti-Xa value of this product was 47.2 units per milligram and the APTT value was 4.6 units per milligram. Thus, the anti-Xa to APTT ratio was determined to be 10.3. This compares with the ratio of 2.8 for the low molecular weight heparin starting material.

The number of ester groups per tetrasaccharide unit contained in the product was indicated to be 1.5 butyryl groups per tetrasaccharide unit.

The infrared spectrum showed an absorption peak at 1736 cm$^{-1}$ for the product. This peak is characteristic of an ester group and was not present in the starting low molecular weight heparin.

The indicated butanol/water partition coefficient for the product was $8.2 \times 10^{-3}$.

The molecular weight of the product was estimated to be 5630.

The permeability of the product was 128 relative to heparin (porcine intestinal mucosa).

EXAMPLE 3 (Comparative)

The procedures of Example 1 were repeated except that heparin (porcine intestinal mucosa) was used instead of the low molecular weight heparin and 40 milliliters of acetyl chloride were used. The product was a dry, white powder.

The product has an anti-Xa value of 79 units per milligram and an APTT value of 6.5 units per milligram. The anti-Xa/APTT ratio was thus found to be 12.2. This compares to the known ratio of 1.0 for heparin.

The number of ester groups per tetrasaccharide unit contained in the product was indicated to be 5.4 acetyl groups per tetrasaccharide unit.

The infrared spectrum of the product showed absorption peak at 1740 cm$^{-1}$. This peak is characteristic of an ester group and was not present the starting heparin.

Using the procedure of Example 1, the butanol/water partition coefficient was indicated to be $0.6 \times 10^{-3}$. This compares with a butanol/water partition coefficient for the heparin starting material of $0.1 \times 10^{-3}$ as determined by the same method.

The molecular weight of the product was estimated to be 16209.

The permeability of the product relative to heparin (porcine intestinal mucosa) was determined by the procedures of Example 1 to be 5.51.

EXAMPLE 4 (Comparative)

The procedures of Example 3 were repeated except that the oil bath was maintained at 50°C and 12 milliliters of propionyl chloride was added instead of the acetyl chloride. The product was a dry, white powder.

The product had an anti-Xa value of 5.4 units per milligram and an APTT value of 6.9 units per milligram. The anti-Xa/APTT ratio was thus found to be 0.78. This compares to the known ratio of 1.0 for heparin.

The number of ester groups per tetrasaccharide unit contained in the product was indicated to be 3.5 propionyl groups per tetrasaccharide unit.

The infrared spectrum showed an absorption peak at 1736 cm$^{-1}$. This peak is characteristic of an ester group and was not present in the starting heparin.

EXAMPLE 5 (Comparative)

The procedures of Example 4 were repeated except that 20 milliliters of propionyl chloride was used. The product was a dry, white powder.

The product had an anti-Xa value of 4.9 units per milligram and an APTT value of 7.1 units per milligram. The anti-Xa/APTT ratio was thus found to be 0.69. This compares to the known ratio of 1.0 for heparin.

The number of ester groups per tetrasaccharide unit contained in the product was indicated to be 2.9 propionyl groups per tetrasaccharide unit.

The infrared spectrum of product showed an absorption peak at 1737 cm$^{-1}$. This peak is characteristic of an ester group and was not present in the starting heparin.

The butanol/water partition coefficient of the product was indicated to be $6.7 \times 10^{-3}$.

The molecular weight of the product was estimated to be 15492.

The permeability of the product relative to heparin (porcine intestinal mucosa) was 63.0.

EXAMPLE 6 (Comparative)

The procedures of Example 5 were repeated except that 6 milliliters of decanoyl chloride was used instead of the propionyl chloride. The product was a dry, white powder.

The product had an anti-Xa value of 21.1 units per milligram and an APTT value of 118.8 units per milligram. The anti-Xa/APTT ratio was thus found to be 0.18. This compares to the known ratio of 1.0 for heparin.

The number of ester groups per tetrasaccharide unit contained in the product was indicated to be 0.2 decanoyl groups per tetrasaccharide unit.

The infrared spectrum of the product showed an absorption peak at 1740 cm$^{-1}$. This peak is characteristic of an ester group and was not present in the starting heparin.

EXAMPLE 7

The procedures of Example 4 were repeated except that low molecular weight heparin was used instead of heparin. The product was a dry, white powder.

Using the procedure of Example 1, the butanol/water partition coefficient of the product was indicated to be 2.0 x 10$^{-3}$.

The number of ester groups per tetrasaccharide unit contained in the product was indicated to be 2.9 propionyl groups per tetrasaccharide unit.

The molecular weight of the product was estimated to be 5872.

The permeability of the product relative to heparin (porcine intestinal mucosa) was 30.6.

The infrared spectrum of the product showed an absorption peak at 1739 cm$^{-1}$. This peak is characteristic of an ester group and was not present in the starting heparin.

EXAMPLE 8

The procedures of Example 2 were repeated except that the oil bath was maintained at 50°C. The product was a dry, white powder.

The indicated butanol/water partition coefficient for the product was 11.0 x 10$^{-3}$.

The number of ester groups per tetrasaccharide unit contained in the product was indicated to be 3.0 butyryl groups per tetrasaccharide unit.

The molecular weight of the product was estimated to be 6073.

The permeability of the product relative to heparin (porcine intestinal mucosa) was 165.

The infrared spectrum of the product showed an absorption peak at 1739 cm$^{-1}$. This peak is characteristic of an ester group and was not present in the starting heparin.

EXAMPLE 9

The procedures of Example 8 were repeated except that 6 milliliters of decanoyl chloride used instead of 20 milliliters of butyryl chloride and the dialysis was conducted against: 95% ethanol for 24 hours, 95% ethanol for 24 hours, 47.5% ethanol for 24 hours, 47.5% ethanol for 24 hours, 1% sodium chloride for 24 hours, 1% sodium chloride for 24 hours, water for 24 hours, and water for 24 hours. The product was a dry, white powder.

The indicated butanol/water partition coefficient of the product was 6.8 x 10$^{-3}$.

The number of ester groups per tetrasaccharide unit contained in the product was 0.36 decanoyl groups per tetrasaccharide unit.

The molecular weight of the product was estimated to be 5421.

The permeability of the product relative to heparin (porcine intestinal mucosa) was 108.

The infrared spectrum of the product showed an absorption peak at 1739 cm$^{-1}$. This peak is characteristic of an ester group and was not present in the starting heparin.

EXAMPLE 10

The procedures of Example 9 were repeated except that 12 milliliters of decanoyl chloride was used. The product was a dry, white powder.

The product had an indicated butanol/water partition coefficient of 44 x 10$^{-3}$.

The number of ester groups per tetrasaccharide unit contained in the product was 0.71 decanoyl groups per tetrasaccharide unit.

The molecular weight of the product was estimated to be 5649.

The permeability of the product relative to heparin (porcine intestinal mucosa) was 685.

The infrared spectrum of the product showed an absorption peak at 1737 cm$^{-1}$. This peak is characteristic of an ester group and was not present in the starting heparin.

EXAMPLE 11

The procedures of Example 10 were repeated except that 24 milliliters of decanoyl chloride was used. The product was a dry, white powder.

The indicated butanol/water partition coefficient of the product was 663 x 10$^{-3}$.

The number of ester groups per tetrasaccharide unit contained in the product was indicated to be 1.03 decanoyl groups per tetrasaccharide unit.

The molecular weight of the product was estimated to be 5857.

The permeability of the product relative to heparin (porcine intestinal mucosa) was 10,100.

The infrared spectrum of the product showed an absorption peak at 1742 cm$^{-1}$. This peak is characteristic of an ester group and was not present in the starting heparin.

EXAMPLE 12 (Comparative)

The procedures of Example 11 were repeated except that heparin (porcine intestinal mucosa) was used instead of low molecular weight heparin. The product was a dry, white powder.

The indicated butanol/water partition coefficient of the product was $215 \times 10^{-3}$.

The number of ester groups per tetrasaccharide unit contained in the product was indicated the presence of 0.9 decanoyl groups per tetrasaccharide unit.

The molecular weight of the product was estimated to be 15227.

The permeability of the product relative to heparin (porcine intestinal mucosa) was 2040.

The infrared spectrum of the product showed an absorption peak at 1740 $cm^{-1}$. This peak is characteristic of an ester group and was not present in the starting heparin.

EXAMPLE 13 (Comparative)

The procedures of Example 8 were repeated except that heparin (porcine intestinal mucosa) was used instead of low molecular weight heparin. The product was a dry, white powder.

The indicated butanol/water partition coefficient of the product was $10.0 \times 10^{-3}$.

The number of ester groups per tetrasaccharide unit contained in the product was indicated to be 2.3 butyryl groups per tetrasaccharide unit.

The molecular weight of the product was estimated to be 15477.

The permeability of the product relative to heparin (porcine intestinal mucosa) was 94.1.

The infrared spectrum of the product showed an absorption peak at 1736 $cm^{-1}$. This peak is characteristic of an ester group and was not present in the starting heparin.

**Claims**
**Claims for the following Contracting States :**
**BE, CH, LI, FR, DE, IT, LU, NL, SE, GB**

1.  An enteral or topical pharmaceutical composition comprising a $C_2$-$C_{10}$ alkanoyl ester obtainable by reacting an acid chloride with a heparin having a molecular weight less than 10,000 daltons and selected from heparin fractions, heparin depolymerization products and anticoagulant mucopolysaccharides synthesized from non-heparin sources, said ester containing more than 0.1 ester groups per tetrasaccharide unit, having a butanol/water (3:2) partition coefficient (as defined in the description) greater than $1 \times 10^{-4}$, having a relative permeability (as defined in the description), as compared to heparin, of 1.5 or greater, and having an anti-Xa/APTT ratio greater than 1.5, and a pharmaceutically acceptable carrier or diluent.

2.  A composition as claimed in Claim 1, wherein the ester groups are acetyl groups.

3.  A composition as claimed in Claim 1, wherein the ester groups are $C_3$-$C_{10}$ alkanoyl groups.

4.  A composition as claimed in Claim 3, wherein the ester groups are propionyl, butyryl or decanoyl groups.

5.  A composition as claimed in any one of the preceding claims, wherein the ester has an anti-Xa/APTT ratio greater than 2.8.

6.  A composition as claimed in any one of the preceding claims, wherein the heparin has a molecular weight less than 6,000 daltons.

7.  An enteral composition as claimed in any one of the preceding claims.

8.  A topical composition as claimed in any one of Claims 1 to 6.

9.  The use of an ester as defined in Claim 1 for the manufacture of a medicament for enteral or topical administration to treat a thromboembolic disorder.

10. A use as claimed in Claim 9, wherein the ester is as defined in any one of Claims 2 to 5.

11. A use as claimed in Claim 9 or Claim 10, wherein the medicament is for enteral administration.

12. A use as claimed in Claim 9 or Claim 10, wherein the medicament is for topical administration.

13. A $C_2$-$C_{10}$ alkanoyl ester obtainable by reacting an acid chloride with a heparin having a molecular weight less than 6,000 daltons and selected from heparin fractions, heparin depolymerization products and anticoagulant mucopolysaccharides synthesized from non-heparin sources, said ester containing more than 0.1 ester groups per tetrasaccharide unit, having a butanol/water (3:2) partition coefficient (as defined in the description) greater than $1 \times 10^{-4}$, having a relative permeability (as defined in the description), as compared to heparin, of 1.5 or greater, and having an anti-Xa/APTT ratio greater than 1.5.

14. An ester as claimed in Claim 13, wherein the ester groups are acetyl groups.

15. An ester as claimed in Claim 13, wherein the ester groups are $C_3$-$C_{10}$ alkanoyl groups.

16. An ester as claimed in Claim 15, wherein the ester groups are propionyl, butyryl or decanoyl groups.

17. An ester as claimed in any one of the preceding claims, wherein the ester has an anti-Xa/APTT ratio greater than 2.8.

18. A pharmaceutically active composition comprising an ester as claimed in Claim 13 and a pharmaceutically acceptable carrier or diluent.

19. A composition as claimed in Claim 18, wherein the ester is as claimed in any one of Claims 14 to 17.

20. The use of an ester as claimed in Claim 13 for the manufacture of a medicament for the treatment of thromboembolic disorders.

21. A use as claimed in Claim 20, wherein the ester is as claimed in any one of Claims 14 to 17.

22. A use of an ester as claimed in Claim 13 for the manufacture of a medicament for the selective inhibition of Factor Xa compared with global anticoagulation activity.

23. A use as claimed in Claim 22, wherein the ester is as claimed in any one of Claims 14 to 17.

**Claims for the following Contracting States : AT, ES, GR**

1. A process for preparing an enteral or topical pharmaceutically active composition comprising mixing with a pharmaceutically acceptable carrier or diluent a $C_2$-$C_{10}$ alkanoyl ester obtainable by reacting an acid chloride with heparin having a molecular weight less than 10,000 daltons and selected from heparin fractions, heparin depolymerization products and anticoagulant mucopolysaccharides synthesized from non-heparin sources, said ester containing more than 0.1 ester groups per tetrasaccharide unit, having a butanol/water (3:2) partition coefficient (as defined in the description) greater than $1 \times 10^{-4}$, having a

relative permeability (as defined in the description), as compared to heparin, of 1.5 or greater, and having an anti-Xa/APTT ratio greater than 1.5.

2. A process as claimed in Claim 1, wherein the ester groups are acetyl groups.

3. A process as claimed in Claim 1, wherein the ester groups are $C_3$-$C_{10}$ alkanoyl groups.

4. A process as claimed in Claim 3, wherein the ester groups are propionyl, butyryl or decanoyl groups.

5. A process as claimed in any one of the preceding claims, wherein the ester has an anti-Xa/APTT ratio greater than 2.8.

6. A process as claimed in any one of the preceding claims, wherein the heparin has a molecular weight less than 6,000 daltons.

7. A process as claimed in any one of the preceding claims, wherein the composition is an enteral composition.

8. A process as claimed in any one of Claims 1 to 6, wherein the composition is a topical composition.

9. A process for preparing a $C_2$-$C_{10}$ alkanoyl ester of a heparin, said ester containing more than 0.1 ester groups per tetrasaccharide unit, having a butanol/water (3:2) partition coefficient (as defined in the description) greater than $1 \times 10^{-4}$, having a relative permeability (as defined in the description), as compared to heparin, of 1.5 or greater, and having an anti-Xa/APTT ratio greater than 1.5, said process comprising reacting an acid chloride with a heparin having a molecular weight less than 6,000 daltons and selected from heparin fractions, heparin depolymerization products and anticoagulant mucopolysaccharides synthesized from non-heparin sources.

10. A process as claimed in Claim 9, wherein the ester groups are acetyl groups.

11. A process as claimed in Claim 9, wherein the ester groups are $C_3$-$C_{10}$ alkanoyl groups.

12. A process as claimed in Claim 11, wherein the ester groups are propionyl, butyryl or decanoyl groups.

**13.** A process as claimed in any one of Claims 9 to 12, wherein the ester has an anti-Xa/APTT ratio greater than 2.8.

**Patentansprüche**
**Patentansprüche für folgende Vertragsstaaten : BE, CH, LI, FR, DE, IT, LU, NL, SE, GB**

**1.** Enterale oder topische pharmazeutische Zusammensetzung, umfassend einen $C_2$-$C_{10}$-Alkanoylester, erhältlich durch Umsetzen eines Säurechlorids mit einem Heparin mit einem Molekulargewicht unter 10000 Dalton, das ausgewählt ist aus Heparin-Fraktionen, Heparin-Depolymerisationsprodukten und antikoagulierend wirkenden Mucopolysacchariden, die aus nicht-Heparin-Quellen synthetisiert worden sind, wobei der Ester mehr als 0,1 Estergruppen pro Tetrasaccharideinheit enthält, einen Butanol/Wasser(3:2)-Verteilungskoeffizienten (wie in der Beschreibung definiert) größer als 1 x $10^{-4}$ aufweist, eine relative Permeabilität (wie in der Beschreibung definiert) im Vergleich zu Heparin von 1,5 oder größer und ein anti-Xa/APTT-Verhältnis größer als 1,5 hat, und einen pharmazeutisch annehmbaren Träger oder ein pharmazeutisch annehmbares Verdünnungsmittel.

**2.** Zusammensetzung nach Anspruch 1, in der die Estergruppen Acetylgruppen sind.

**3.** Zusammensetzung nach Anspruch 1, in der die Estergruppen $C_3$-$C_{10}$-Alkanoylgruppen sind.

**4.** Zusammensetzung nach Anspruch 3, in der die Estergruppen Propionyl-, Butyryl- oder Dekanoylgruppen sind.

**5.** Zusammensetzung nach irgendeinem der vorangegangenen Ansprüche, in der der Ester ein anti-Xa/APTT-Verhältnis größer als 2,8 hat.

**6.** Zusammensetzung nach irgendeinem der vorangegangenen Ansprüche, in der das Heparin ein Molekulargewicht von weniger als 6000 Dalton aufweist.

**7.** Enterale Zusammensetzung nach irgendeinem der vorangegangenen Ansprüche.

**8.** Topische Zusammensetzung nach irgendeinem der Ansprüche 1 bis 6.

**9.** Verwendung eines wie in Anspruch 1 definierten Esters zur Herstellung eines Arzneimittels für eine enterale oder topische Verabreichung, um eine thromboembolische Erkrankung zu behandeln.

**10.** Verwendung nach Anspruch 9, in der der Ester wie in irgendeinem der Ansprüche 2 bis 5 definiert ist.

**11.** Verwendung nach Anspruch 9 oder 10, in der das Arzneimittel für eine enterale Verabreichung bestimmt ist.

**12.** Verwendung nach Anspruch 9 oder 10, in der das Arzneimittel für eine topische Verabreichung bestimmt ist.

**13.** $C_2$-$C_{10}$-Alkanoylester, erhältlich durch Umsetzen eines Säurechlorids mit einem Heparin mit einem Molekulargewicht unter 6000 Dalton, das ausgewählt ist aus Heparin-Fraktionen, Heparin-Depolymerisationsprodukten und antikoagulierend wirkenden Mucopolysacchariden, die aus nicht-Heparin-Quellen synthetisiert worden sind, wobei der Ester mehr als 0,1 Estergruppen pro Tetrasaccharideinheit enthält, einen Butanol/Wasser (3:2)-Verteilungskoeffizienten (wie in der Beschreibung definiert) größer als 1 x $10^{-4}$ aufweist, eine relative Permeabilität (wie in der Beschreibung definiert) im Vergleich zu Heparin von 1,5 oder größer und ein anti-Xa/APTT-Verhältnis größer als 1,5 hat.

**14.** Ester nach Anspruch 13, in dem die Estergruppen Acetylgruppen sind.

**15.** Ester nach Anspruch 13, in dem die Estergruppen $C_3$-$C_{10}$-Alkanoylgruppen sind.

**16.** Ester nach Anspruch 15, in dem die Estergruppen Propionyl-, Butyryl- oder Dekanoylgruppen sind.

**17.** Ester nach irgendeinem der vorangegangenen Ansprüche, wobei der Ester ein anti-Xa/APTT-Verhältnis größer als 2,8 hat.

**18.** Pharmazeutisch wirksame Zusammensetzung umfassend einen Ester nach Anspruch 13 und einen pharmazeutisch annehmbaren Träger oder ein pharmazeutisch annehmbares Verdünnungsmittel.

**19.** Zusammensetzung nach Anspruch 18, in der der Ester ein Ester nach irgendeinem der Ansprüche 14 bis 17 ist.

**20.** Verwendung eines Esters nach Anspruch 13 zur Herstellung eines Arzneimittels für die Behandlung thromboembolischer Erkrankungen.

**21.** Verwendung nach Anspruch 20, bei der der Ester ein Ester nach irgendeinem der Ansprüche 14 bis 17 ist.

**22.** Verwendung eines Esters nach Anspruch 13 zur Herstellung eines Arzneimittels für die selektive Hemmung von Faktor Xa im Vergleich zu globaler antikoagulierender Aktivität.

**23.** Verwendung nach Anspruch 22, bei der der Ester ein Ester nach irgendeinem der Ansprüche 14 bis 17 ist.

**Patentansprüche für folgende Vertragsstaaten : AT, ES, GR**

**1.** Verfahren zur Herstellung einer enteralen oder topischen, pharmazeutisch wirksamen Zusammensetzung, das umfaßt, mit einem pharmazeutisch annehmbaren Träger oder Verdünnungsmittel einen $C_2$-$C_{10}$-Alkanoylester, erhältlich durch Umsetzen eines Säurechlorids mit einem Heparin mit einem Molekulargewicht unter 10000 Dalton, das ausgewählt ist aus Heparin-Fraktionen, Heparin-Depolymerisationsprodukten und antikoagulierend wirkenden Mucopolysacchariden, die aus nicht-Heparin-Quellen synthetisiert worden sind, wobei der Ester mehr als 0,1 Estergruppen pro Tetrasaccharideinheit enthält, einen Butanol/Wasser (3:2)-Verteilungskoeffizienten (wie in der Beschreibung definiert) größer als $1 \times 10^{-4}$ aufweist, eine relative Permeabilität (wie in der Beschreibung definiert) im Vergleich zu Heparin von 1,5 oder größer und ein anti-Xa/APTT-Verhältnis größer als 1,5 hat, zu mischen.

**2.** Verfahren nach Anspruch 1, wobei die Estergruppen Acetylgruppen sind.

**3.** Verfahren nach Anspruch 1, wobei die Estergruppen $C_3$-$C_{10}$-Alkanoylgruppen sind.

**4.** Verfahren nach Anspruch 3, wobei die Estergruppen Propionyl-, Butyryl- oder Dekanoylgruppen sind.

**5.** Verfahren nach irgendeinem der vorangegangenen Ansprüche, wobei der Ester ein anti-Xa/APTT-Verhältnis größer als 2,8 hat.

**6.** Verfahren nach irgendeinem der vorangegangenen Ansprüche, wobei das Heparin ein Molekulargewicht unter 6000 Dalton aufweist.

**7.** Verfahren nach irgendeinem der vorangegangenen Ansprüche, wobei die Zusammensetzung eine enterale Zusammensetzung ist.

**8.** Verfahren nach irgendeinem der Ansprüche 1 bis 6, wobei die Zusammensetzung eine topische Zusammensetzung ist.

**9.** Verfahren zum Herstellen eines $C_2$-$C_{10}$-Alkanoylester eines Heparins, wobei der Ester mehr als 0,1 Estergruppen pro Tetrasacccharideinheit enthält, einen Butanol/Wasser (3:2)-Verteilungskoeffizienten (wie in der Beschreibung definiert) größer als $1 \times 10^{-4}$ aufweist, eine relative Permeabilität (wie in der Beschreibung definiert) im Vergleich zu Heparin von 1,5 oder größer und ein anti-Xa/APTT-Verhältnis größer als 1,5 hat, wobei das Verfahren umfaßt, ein Säurechlorid mit einem Heparin mit einem Molekulargewicht unter 6000 Dalton, das ausgewählt ist aus Heparin-Fraktionen, Heparin-Depolymerisationsprodukten und antikoagulierend wirkenden Mucopolysacchariden, die aus nicht-Heparin-Quellen synthetisiert worden sind, umzusetzen.

**10.** Verfahren nach Anspruch 9, wobei die Estergruppen Acetylgruppen sind.

**11.** Verfahren nach Anspruch 9, wobei die Estergruppen $C_3$-$C_{10}$-Alkanoylgruppen sind.

**12.** Verfahren nach Anspruch 11, wobei die Estergruppen Propionyl-, Butyryl- oder Dekanoylgruppen sind.

**13.** Verfahren nach irgendeinem der Ansprüche 9 bis 12, wobei der Ester ein anti-Xa/APTT-Verhältnis größer als 2,8 hat.

**Revendications**
**Revendications pour les Etats contractants suivants : BE, CH, LI, FR, DE, IT, LU, NL, SE, GB**

**1.** Composition pharmaceutique entérale ou topique comprenant un ester alkanoyle en $C_2$-$C_{10}$ pouvant être obtenu par mise en réaction d'un chlorure d'acide avec une héparine ayant une masse moléculaire inférieure à 10.000 daltons et choisie parmi les fractions d'héparine, les produits de dépolymérisation d'héparine et des mucopolysaccharides anticoagulants synthétisés à partir de sources non héparines, l'ester contenant plus de 0,1 groupe ester par unité tétrasaccharide, ayant un coefficient de séparation butanol/eau (3:2) (tel que défini dans la description) supérieur à $1 \times 10^{-4}$, ayant une perméabilité relative (telle que définie dans la description), par rapport à l'héparine, de 1,5 ou supérieure, et ayant un rapport anti-Xa/APTT supérieur à 1,5, et un porteur ou diluant acceptable pharmaceutiquement.

**2.** Composition selon la revendication 1, dans laquelle les groupes ester sont des groupes acétyle.

**3.** Composition selon la revendication 1, dans laquelle les groupes ester sont des groupes alkanoyle $C_3$-$C_{10}$.

**4.** Composition selon la revendication 3, dans laquelle les groupes ester sont des groupes propionyle, butyryle ou décanoyle.

**5.** Composition selon l'une quelconque des revendications précédentes, dans laquelle l'ester a un rapport anti-Xa/APTT supérieur à 2,8.

**6.** Composition selon l'une quelconque des revendications précédentes, dans laquelle l'héparine a une masse moléculaire inférieure à 6.000 daltons.

**7.** Composition entérale selon l'une quelconque des revendications précédentes.

**8.** Composition topique selon l'une quelconque des revendications 1 à 6.

**9.** Utilisation d'un ester tel que défini dans la revendication 1 pour la fabrication d'un médicament destiné à l'administration entérale ou topique pour traiter un trouble thromboembolique.

**10.** Utilisation selon la revendication 9, dans laquelle l'ester est tel que défini dans l'une quelconque des revendications 2 à 5.

**11.** Utilisation telle que revendiquée dans la revendication 9 ou la revendication 10, dans laquelle le médicament est destiné à l'administration entérale.

**12.** Utilisation selon la revendication 9 ou la revendication 10, dans laquelle le médicament est destiné à l'administration topique.

**13.** Ester alkanoyle en $C_2$-$C_{10}$ pouvant être obtenu par mise en réaction d'un chlorure d'acide avec une héparine ayant une masse moléculaire inférieure à 6000 daltons et choisi parmi les fractions d'héparine, des produits de dépolymérisation d'héparine et des mucopolysaccharides anticoagulants synthétisés à partir de sources non héparines, l'ester contenant plus de 0,1 groupe ester par unité tetrasaccharide, ayant un coefficient de séparation butanol/eau (3:2) (tel que défini dans la description) supérieur à 1 X $10^{-4}$, ayant une perméabilité relative (telle que définie dans la description), par rapport à l'héparine, de 1,5 ou supérieure et ayant un rapport anti Xa/APTT supérieur à 1,5.

**14.** Ester selon la revendication 13, dans laquelle les groupes ester sont des groupes acétyle.

**15.** Ester selon la revendication 13, dans laquelle les groupes ester sont des groupes alkanoyle en $C_3$-$C_{10}$.

**16.** Ester selon la revendication 15, dans laquelle les groupes ester sont des groupes propionyle, butyryle ou décanoyle.

**17.** Ester selon l'une quelconque des revendications précédentes, dans lequel l'ester possède un rapport anti-Xa/APTT supérieur à 2,8.

**18.** Composition active pharmaceutiquement comprenant un ester selon la revendication 13 et un porteur ou diluant acceptable pharmaceutiquement.

**19.** Composition selon la revendication 18, dans lequel l'ester est tel que revendiqué dans l'une quelconque des revendications 14 à 17.

**20.** Utilisation d'un ester tel que revendiqué dans la revendication 13 pour la fabrication d'un médicament destiné au traitement de troubles thromboemboliques.

**21.** Utilisation telle que revendiquée dans la revendication 20, dans laquelle l'ester est tel que revendiqué dans l'une quelconque des revendications 14 à 17.

**22.** Utilisation d'un ester selon la revendication 13 pour la fabrication d'un médicament destiné à l'inhibition sélective du facteur Xa par rapport à l'activité d'anticoagulation globale.

**23.** Utilisation selon la revendication 22, dans laquelle l'ester est tel que revendiqué dans l'une quelconque des revendications 14 à 17.

**Revendications pour les Etats contractants suivants : AT, ES, GR**

**1.** Procédé pour la préparation d'une composition entérale ou topique active pharmaceutiquement comprenant le mélange avec un support ou un diluant acceptable pharmaceutiquement, un ester alkanoyle $C_2$-$C_{10}$ pouvant être obtenu par mise en réaction d'un chlorure d'acide avec une héparine ayant une masse moléculaire inférieure à 10.000 daltons et choisie parmi les fractions d'héparine, les produits de dépolymérisation d'héparine et des mucopolysaccharides anticoagulants synthétisés à partir de sources non héparines, l'ester contenant plus de 0,1 groupe ester par unité tetrasaccharide, ayant un coefficient de séparation butanol/eau (3:2) (tel que défini dans la description) supérieur à 1 X 10$^{-4}$, ayant une perméabilité relative (telle que définie dans la description), par rapport à l'héparine, de 1,5 ou supérieure, et ayant un rapport anti-Xa/APTT supérieur à 1,5.

2. Procédé selon la revendication 1, dans lequel les groupes ester sont des groupes acétyles.

3. Procédé selon la revendication 1, dans lequel les groupes ester sont des groupes alkanoyle en $C_3$-$C_{10}$.

4. Procédé selon la revendication 3, dans lequel les groupes ester sont des groupes propionyle, butyryle ou décanoyle.

5. Procédé selon l'une quelconque des revendications précédentes, dans lequel l'ester a un rapport anti-Xa/APTT supérieur à 2,8.

6. Procédé selon l'une quelconque des revendications précédentes, dans lequel l'héparine a une masse moléculaire inférieure à 6.000 daltons.

7. Procédé selon l'une quelconque des revendications précédentes, dans lequel la composition est une composition entérale.

8. Procédé selon l'une quelconque des revendications 1 à 6, dans lequel la composition est une composition topique.

9. Procédé pour la préparation d'un ester alkanoyle en $C_2$-$C_{10}$ d'une héparine, l'ester contenant plus de 0,1 groupe ester par unité tétrasaccharide, ayant un coefficient de séparation butanol/eau (3:2) (tel que défini dans la description) supérieur à 1 X 10$^{-4}$, ayant une perméabilité relative (telle que définie dans la description), comparée à l'héparine, de 1,5 ou supérieure, et ayant un rapport anti-Xa/APTT supérieur à 1,5, le procédé comprenant la mise en réaction d'un chlorure d'acide avec une héparine ayant un poids moléculaire inférieur à 6000 daltons et choisie à partir de fractions d'héparine, les produits de dépolymérisation d'héparine et des mucopolysaccharides anticoagulants synthétisés à partir de sources non héparines.

10. Procédé selon la revendication 9, dans lequel les groupes ester sont des groupes acétyle.

11. Procédé selon la revendication 9, dans lequel les groupes ester sont des groupes alkanoyle en $C_3$-$C_{10}$.

12. Procédé selon la revendication 11, dans lequel les groupes ester sont des groupes propionyle, butyryle ou décanoyle.

13. Procédé selon l'une quelconque des revendications 9 à 12, dans lequel l'ester a un rapport anti-Xa/APTT supérieur à 2,8.